# EUROPEAN PATENT SPECIFICATION

(11) **EP 1 353 692 B1**
(45) Date of publication and mention of the grant of the patent: **25.03.2009**
(21) Application number: 01990804.5
(22) Date of filing: 01.11.2001
(51) Int. Cl.: A61K 41/00, A61K 31/495, A61P 27/02

(54) **METHODS AND COMPOSITIONS FOR TREATMENT OF OCULAR NEOVASCULARIZATION AND NEURAL INJURY**
VERFAHREN UND ZUSAMMENSETZUNGEN ZUR BEHANDLUNG VON OKULARER NEOVASKULARISIERUNG UND NERVENVERLETZUNGEN
COMPOSITIONS ET METHODES DE TRAITEMENT DE LA NEOVASCULARISATION OCULAIRE ET DES LESIONS NERVEUSES

(30) Priority: 01.11.2000 US 244850 P
(43) Date of publication of application: 22.10.2003
(73) Proprietor: ALLERGAN, INC., Irvine CA 92612 (US)
(72) Inventor: BURKE, James, A., Santa Ana, CA 92705 (US); LIN, Ton, Irvine, CA 92602 (US); WHEELER, Larry, A., Irvine, CA 92715 (US); DE VRIES, Gerald, W., Laguna Hills, CA 92653 (US)
(74) Representative: HOFFMANN EITLE
(86) International application number: PCT/US2001/046014
(87) International publication number: WO 2002/036162

(56) References cited:
- WO-A-00/40245
- WO-A-02/64163

## Description

This application claims priority pursuant to 35 USC 119 to provisional application Serial No. 60/244,850, filed Nov. 1, 2000.

### Background Of The Invention

Loss of visual acuity is a common problem associated with aging and with various conditions of the eye. Particularly troublesome is the development of unwanted neovascularization in the cornea, retina or choroid. Choroidal neovascularization leads to hemorrhage and fibrosis, with resultant visual loss in a number of recognized eye diseases, including macular degeneration, ocular histoplasmosis syndrome, myopia, diabetic retinopathy and inflammatory diseases.

Age-related macular degeneration (AMD) is the leading cause of new blindness in the elderly, and choroidal neovascularization is responsible for 80% of the severe visual loss in patients with this disease. Although the natural history of the disease is eventual quiescence and regression of the neovascularization process, this usually occurs at the cost of sub-retinal fibrosis and vision loss.

Traditional treatment of AMD relies on occlusion of the blood vessels using laser photocoagulation. However, such treatment requires thermal destruction of the neovascular tissue, and is accompanied by full-thickness retinal damage, as well as damage to medium and large choroidal vessels. Further, the subject is left with an atrophic scar and visual scotoma. Moreover, recurrences are common, and visual prognosis is poor.

Recently, forms of photocoagulation have been devised which attempt to reduce the damage attendant to traditional photocoagulation. For example, transpupillary thermotherapy (TTT) utilizes a low intensity laser in combination with a large "spot" (irradiation focal point) size and long exposure to close choroidal neovascularization and thereby treat macular degeneration. This procedure is said to reduce the amount of secondary damage seen in the use of traditional photocoagulation procedures.

### Photodynamic Therapy

Recent research in the treatment of neovascularization have had the aim of causing more selective closure of the blood vessels, in order to preserve the overlying neurosensory retina. One such strategy is a treatment termed photodynamic therapy or PDT, which relies on low intensity light exposure of photosensitized tissues to produce lesions in the newly developing blood vessels. In PDT, photoactive compounds are administered and allowed to reach a particular undesired tissue which is then irradiated with a light absorbed by the photoactive compound. This results in destruction or impairment of the tissue immediately surrounding the locus of the photoactive compound without the more extensive ocular tissue damage seen when photocoagulation is used.

Photodynamic therapy of conditions in the eye has been attempted over the past several decades using various photoactive compounds, e.g., porphyrin derivatives, such as hematoporphyrin derivative and Photofrin porfimer sodium; "green porphyrins", such as benzoporphyrin derivative (BPD), MA; and phthalocyanines. Photodynamic treatment of eye conditions has been reported to actually enhance the visual acuity of certain subjects in some circumstances. U.S. Patent No. 5,756,541.

However, although generally more safe than photocoagulation, there are certain dangers involved in performing PDT. For example, low intensity lasers in conjunction with the systemic injection of vertporfin is currently the only approved PDT for treatment of age-related macular degeneration. But studies have shown that the use of vertporfin at high doses (12 and 18 mg/m²) result in long term or permanent scarring of the retina, chronic absence of photoreceptor cells, and optic nerve atrophy. Reinke et al., Ophthalmology 106:1915 (October 1999), incorporated by reference herein. At lower concentrations of vertporfin (e.g., about 6 mg/m²) PDT is effective to slow vascular outgrowth and associated edema somewhat, but treatment appears to be necessary every few months.

Additionally, while PDT is clearly efficacious in some patients, this mode of treatment has resulted in a lower percentage of patients reporting an increase in visual acuity, or a halting in the progression of visual deterioration, than would be expected theoretically. The reasons for this have not been clearly understood in the literature, but may relate to PDT-induced neurosensory damage which limits efficacy.

### The Alpha Adrenergic Receptors

Human adrenergic receptors are integral membrane proteins which have been classified into two broad classes, the alpha and the beta adrenergic receptors. Both types mediate the action of the peripheral sympathetic nervous system upon binding of catecholamines, norepinephrine and epinephrine.

Norepinephrine is produced by adrenergic nerve endings, while epinephrine is produced by the adrenal medulla. The binding affinity of adrenergic receptors for these compounds forms one basis of the classification: alpha receptors tend to bind norepinephrine more strongly than epinephrine and much more strongly than the synthetic compound isoproterenol. The preferred binding affinity of these hormones is reversed for the beta receptors. In many tissues, the functional responses, such as smooth muscle contraction, induced by alpha receptor activation are opposed to responses induced by beta receptor binding.

Subsequently, the functional distinction between alpha and beta receptors was further highlighted and refined by the pharmacological characterization of these receptors from various animal and tissue sources. As a result, alpha and beta adrenergic receptors were further subdivided into α₁, α₂, β₁, and β₂ subtypes.

Functional differences between α₁ and α₂ receptors have been recognized, and compounds which exhibit selective binding between these two subtypes have been developed. Thus, in WO 92/0073, the selective ability of the R(+) enantiomer of terazosin to selectively bind to adrenergic receptors of the α₁ subtype was reported. The α₁/α₂ selectivity of this compound was disclosed as being significant because agonist stimulation of the α₂ receptors was said to inhibit secretion of epinephrine and norepinephrine, while antagonism of the α₂ receptor was said to increase secretion of these hormones. Thus, the use of non-selective alpha-adrenergic blockers, such as phenoxybenzamine and phentolamine, was said to be limited by their induction, through the α₂ adrenergic receptors, of increased concentrations of plasma catecholamine and attendant physiological sequelae (increased heart rate and smooth muscle contraction).

For a general background on the α-adrenergic receptors', the reader's attention is directed to Robert R. Ruffolo, Jr., α-Adrenoreceptors: Molecular Biology, Biochemistry and Pharmacology, (Progress in Basic and Clinical Pharmacology series, Karger, 1991), incorporated by reference herein, in which the basis of α₁/α₂ subclassification, the molecular biology, signal transduction, agonist structure-activity relationships, receptor functions, and therapeutic applications for compounds exhibiting α-adrenergic receptor affinity is explored.

The cloning, sequencing and expression of alpha receptor subtypes from animal tissues has led to the subclassification of the α₁ adrenoreceptors into the further classifications of α_{1A}, α_{1B}, and α_{1D}. Similarly, the at adrenoreceptors have also been classified α_{2A}, α_{2B}, and α_{2C} receptors. Each α₂ receptor subtype appears to exhibit its own pharmacological and tissue specificities. Compounds having a degree of specificity for one or more of these subtypes may be more specific therapeutic agents for a given indication than the currently employed α₂ receptor pan-agonists (such as the drugs clonidine and brimonidine).

WO 00/40245 discloses brimonidine for protecting the retina from damage by laser light during the treatment of ARMD, in particular neovascular ARMD. It is mentioned that brimonidine has a neuroprotective effect. Brimonidine is disclosed to have a quinoxaline core structure and to be a "relatively selective α₂ agonist".

### BRIEF DESCRIPTION OF THE DRAWINGS

Figure 1A shows that brimonidine inhibits the post PDT decrease in retinal functionality as compared to a saline control.
Figure 1B shows that brimonidine does not affect the area of ablation of CNV following PDT.
Figure 2B shows that Alphagar^{®} (brimonidine) has no effect on retinal thickness in non-PDT-treated rabbit eyes.

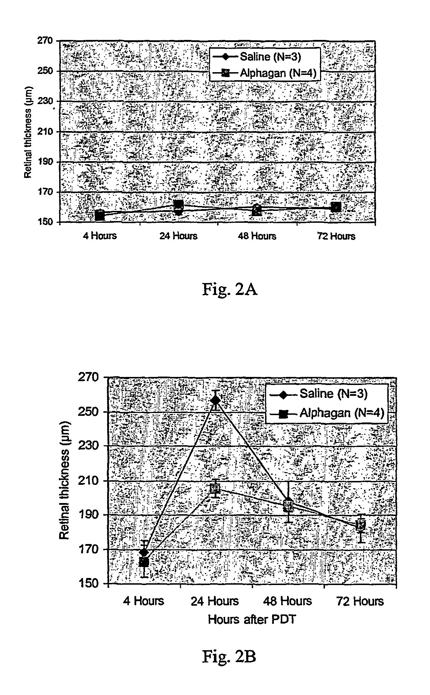

Figure 2B shows that brimonidine decreases the increase in retinal thickness (edema) appearing after PDT treatment.

### SUMMARY OF THE INVENTION

The present invention is directed to the use of an a receptor agonist for the manufacture of a medicament for use in a method for reducing or eliminating a decrease in neurosensory retinal function following laser treatment of chorodial neovanscularization (CNV) while maintaining the vascular occlusion therapeutic effect of such therapy, the method comprising the steps: a) administering to a mammal having a CNV a therapeutically effective amount of an alpha receptor agonist, b) subjecting said mammal to laser irradiation of the retinal locus of the CNV; wherein the amount of neurosensory retinal function following steps a) and b) is greater than when said mammal is subjected to step b) without step a). In a preferred embodiment, the mammal is given a therapeutically effective amount of a pharmaceutically acceptable photoactivated dye capable of accumulation in the locus of a choroidal neovascularization and destroying tissue when exposed to light of the same wavelength as the laser.

In these methods, the alpha adrenergic receptor agonist is selected from the groups consisting of:
an alpha 2B, alpha 2C selective agonist and clonidine.

Other alpha 2B selective compounds include AGN 960, AGN 795 and AGN 923. The structure of AGN 960 is presented elsewhere in this patent application. The structure of AGN 795 is as follows:

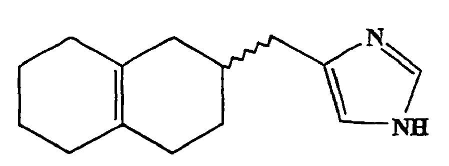

The structure of AGN 923 is as follows:

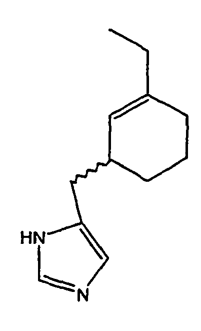

By "effective amount" of a neuroprotective agent (such as an alpha adrenergic agonist) is meant an amount effective to reduce the amount of cell death among the neurons of the retina and optic nerve (e.g., photoreceptors, retinal ganglion cells, and bipolar cells, or any of these) caused by the photoactive component of laser treatment as compared to a similarly situated CNV patient receiving laser treatment who does not receive treatment with the neuroprotective agent.

When an alpha adrenergic agonist or another agent having neuroprotective activity is used in conjunction with PDT or photocoagulation, it is preferred that the amount of such agent administered to the patient is an effective neuroprotective dose.

Determining the absolute dosage of the neuroprotective agent depends upon a number of factors, including the means of administration and delivery and the form of the drug. For intraocular delivery agent, such as by intravitreal or subretinal injection, dosages are preferably in the range of about 0.1 ug to about 100 ug per eye; more preferably in the range of about 0.20 ug to about 50 ug per eye; even more preferably in the range of about 0.5 ug to about 10 ug per eye.

The neuroprotective agent may be delivered by any means effective to expose the retinal and optic nerve cells to the agent. Thus, such agents may be delivered systemically, such as by intravenous, intramuscular, or subcutaneous injection, or by oral delivery. Alternatively, the neuroprotective and/or neovascularization-inhibiting agent (s) may be delivered by direct injection into the eye, such as into the anterior chamber, posterior chamber or vitreous chamber, or by subretinal injection. The reagent may also be delivered topically to the ocular surface.

Another delivery method provides for sustained delivery of the noeuroprotective agent using an intraocular implant. Such implants may be, for example, a biodegradable and/or biocompatible implant or insert such as the ocular implants and inserts disclosed in United States Patents No. 5,443,505, 5,824,072, 5,766,242; 4,853,224; 4,997,652; 5,164,188; 5,632,984; and 5,869,079, incorporated by reference herein. Such implants may be inserted into a chamber of the eye, such as the anterior, posterior or anterior chambers, or may be implanted in the sclera, transchoroidal space, or an avascularized region exterior to the vitreous.

### DETAILED DESCRIPTION OF THE INVENTION

The present invention is drawn to the manufacture of a methods medicament for therapeutic for the treatment of intraocular neovascularization associated with conditions such as age-related macular degeneration (ARMD), diabetic retinopathy, ocular histoplasmosis syndrome, and pathologic myopia.

Said therapeutic methods combine retinal photocoagulation or photodynamic therapy (PDT) with a neuroprotectant agent, which is an alpha adrenergic agonist. The agent is an alpha 2B and/or alpha 2C selective agonist. One can also combine the neuroprotective alpha adrenergic agonist with other neuroprotective agents to take advantage of different mechanistic means of providing neuroprotection.

In a preferred aspect of this embodiment of the invention, the neuroprotective agent is administered to the patient sufficiently prior to photcoagulation (such as TTT) or PDT treatment so as to be available to protect nerve cells upon the commencement of therapy. In another aspect of the invention, the alpha adrenergic receptor agonist is administered a sufficient time following photocoagulation or PDT treatment to forestall nerve death due to such treatment.

Such methods are applicable to any photocoagulation method or to PDT treatment which makes use of any photoactive compound. Such photoactive compounds may include derivatives of hematoporphyrin, as described in U.S. Patents No. 5,028,621; 4,866,168; 4,649,151; and 5,438,071. pheophorbides are described in U.S.Pat. Nos. 5,198,460; 5,002,962; and 5,093,349; bacteriochlorins in U.S. Pat. Nos. 5,171,741 and 5,173,504; dimers and trimers of hematoporphyrins in U.S. Pat. Nos, 4,968,715 and 5,190,966. Other possible photoactive compounds include purpurins, merocyanines and porphycenes. All of the aforementioned patents are incorporated by reference herein. Of course, mixtures of photoactive compounds may be used in conjunction with each other.

A currently preferred photoactive compound is verteporfin (liposomal benzoporphyrin derivative). This compound is currently the only photoactive agent approved by the U.S. Food and Drug Administration for treatment of choroidal neovascularization in conjunction with photodynamic therapy.

The photoactive agent is formulated so as to provide an effective concentration to the target ocular tissue. The photoactive agent may be coupled to a specific binding ligand which may bind to a specific surface component of the target ocular tissue, such as a cell surface receptor or, if desired, may be formulated with a carrier that delivers higher concentrations of the photoactive agent to the target tissue. Exemplary ligands may be receptor antagonists or a variable region of an immunoglobulin molecule.

The nature of the formulation will depend in part on the mode of administration and on the nature of the photoactive agent selected. Any pharmaceutically acceptable excipient, or combination thereof, appropriate to and compatible with the particular photoactive compound may be used, Thus, the photoactive compound may be administered as an aqueous composition, as a transmucosal or transdermal composition, or in an oral formulation. The formulation may also include liposomes. Liposomal compositions are particularly preferred especially where the photoactive agent is a green porphyrin. Liposomal formulations are believed to deliver the green porphyrin with a measure of selectivity to the low-density lipoprotein component of plasma which, in turn acts as a carrier to deliver the active ingredient more effectively to the desired site. Increased numbers of LDL receptors have been shown to be associated with neovascularization, and by increasing the partitioning of the green porphyrin into the lipoprotein phase of the blood, it appears to be delivered more efficiently to neovasculature.

Consistent with the chosen formulation, the photoactive compound may be delivered in a variety of ways. For example, delivery may be oral, peritoneal, rectal, or topical (e.g., by installation directly into the eye). Alternatively, delivery may be by intravenous, intramuscular or subcutaneous injection.

The dosage of the photoactive compound may vary, according to the activity of the specific compound(s) chosen, the formulation, and whether the compound is joined to a carrier and thus targeted to a specific tissue as described above. When using green porphyrins, dosages are usually in the range of 0.1-50 mg/M² of body surface area; more preferably from about 1-10 mg/M² or from about 2-8 mg/M². Obviously, parameters to be considered when determining the dosage include the duration and wavelength of the light irradiation, the nature of the photochemical reaction induced by the light irradiation, and the dye-to-laser time period.

Light irradiation is performed a sufficient time after the administration of the photoactive compound so as to permit the compound to reach its target tissue. Upon being irradiated with the wavelength(s) appropriate to the compound(s) chosen, the compound enters an excited state and is thought to interact with other compounds to form highly reactive intermediates which can then destroy the target endothelial tissue, causing platelet aggregation and thrombosis. Fluence of the irradiation may vary depending on factors such as the depth of tissue to be treated and the tissue type - generally it is between about 25 and about 200 Joules/cm². Irradiance typically is between about 150 and about 900 mW/cm², but can also vary somewhat from this range.

Typically, light treatment is given about 5 minutes following administration of the photoactive drug. In a preferred embodiment, the photoactive drug is administered intravenously.

The other component(s) of the methods the present invention are a relating to neuroprotective alpha adrenergic agent. Neuroprotective agents are clonidine and alpha 2B and or alpha 2C selective agonists such as, without limitation, those mentioned in US Patent No. 6,313,172 and patent publications WO0178703, WO0178702, WO0100586 and WO9928300. These patents and patent applications are owned by the assignee of the present patent application, and are hereby incorporated by reference herein.

In a most preferred embodiment of the invention, the compound is an alpha 2B selective agonist.

Brimonidine has been reported to have neuroprotective activity. Hence, US Patent No. 5,856,329 and Yoles E., et al., Invest. Ophthalmol. Vis. Sci. 40: 65 (1999)disclose this property of brimonidine; however, neither of these references makes any suggestion that brimonidine be used in the treatment of CNV.

The neuroprotective alpha adrenergic agent(s) of the present invention are delivered in any manner in which it is effective to protect neurons and/or inhibit neovascularization incident to photocoagulation or PDT treatment. Generally, the agent(s) is administered prior to laser treatment, so as to permit it to reach the ocular neural tissue before phototherapy. This will permit the agent(s) to have an immediate protective effect on neural cells. However, the neovascularization-inhibiting benefits of such an agent can be realized even when given simultaneously with, or shortly after PDT treatment.

Additionally, the alpha adrenergic agents useful in the present method may be joined, in a manner similar to that of the photoactive compounds, to cell surface targeting ligands, such as portions of an antibody or immunologically active fragments to aid in targeting the drug to ocular cells, such as the optic nerve neurons and photoreceptors.

The neuroprotective alpha agonist agent may be formulated for oral delivery in, for example, a capsule, tablet or liquid, or for intravenous, intramuscular, or subcutaneous injection. In such a formulation, any suitable excipient may be added to such a formulation to stabilize the active ingredient and, particularly in the case of intravenous administration, to provide the necessary electrolyte balance. The alpha adrenergic agonists used in the methods of the present invention may also be formulated as a suppository or otherwise administered rectally. Formulations appropriate for rectal drug administration are well-known to those of skill in the art.

In yet another embodiment the alpha adrenergic agonist agents may be formulated within liposomes. The liposomes are then able to fuse with a cell membrane, thus delivering the nucleic acid to receptors located at the cell surface and within the cell.

As indicated above, the neuroprotective agents to be used in the present invention may be administered by systemic delivery, as by intravenous, intramuscular or subcutaneous injection. In addition, these agents may be delivered directly to the eye by biocompatable and/or biodegradable implants or inserts (such as those described in patents cited and incorporated by preference above) containing the drug, or by direct injection into the eye, for example by intravitreal and/or subretinal injection. Alternatively, the alpha adrenergic agents may be topically applied to the surface in an drop.

The therapeutically effective dosage of such agents will depend upon factors including the mode of delivery, the specific activity of the polypeptide, and the formulation in which the agent is fabricated. Once a formulation and route of administration is decided upon, determining a therapeutically effective dose is routine in the pharmaceutical arts, and can be readily determined without undue experimentation using suitable animal models such as, without limitation, non-human primates and rabbits.

Preferably, the dosage regimen of the neuroprotective agent will be such to permit the active agent to remain in contact with retinal cells throughout the treatment period. Thus, the agent may be administered, for example, once or twice a day for 12 weeks.

### Example 1

A 74 year old patient presents with "wet" age-related macular degeneration (ARMD) in the foveal region of the right eye, and his condition is found to be suitable for photodynamic therapy (PDT). For one week prior to the date of scheduled treatment, the patient is given a topical dosage of brimonidine twice a day in a standard formulation.

The day of scheduled PDT treatment, the patient is administered 6 mg/M² of verteporfin. Fifteen minutes after the start of the infusion, the patient is administered Irradiance of 600 mW/cm² and total fluence of 50 Joules/cm² from an Argon light laser.

Brimonidine administration is continued every two days throughout the 12 week evaluation period.

Evaluation of neural health is assayed 1 week, 4 weeks, and 12 weeks following treatment by visual inspection of the retina and test of visual acuity. The affected areas of the retina appear healthy with no whitening (indicating lack of discernable retina damage) or edema one week following PDT treatment; this trend continues throughout the monitoring period. Fluorescein angiography at same time points shows minimal leakage in the treated tissue after one week, and this minimal leakage continues throughout the monitoring period. No evidence of renewed neovascularization can be seen 12 weeks following PDT treatment. Additionally, no evidence of optic nerve axon loss can been seen. Tests of visual acuity 4 and 12 weeks following combined PDT and PEDF treatment show no discernable loss of vision, as a result of the treatment.

### Example 2

Same facts as in Example 1, except that rather than being given topical brimonidine, the affected eye is given an biodegradable intraocular implant, by injection into the vitreous humor. The implant placed in the eye by intravitreal injection three days prior to PDT treatment ; and is readministered 10 days following PDT treatment. The retina is examined following the 12 week evaluation period.

Evaluation of neural health is assayed 1 week, 4 weeks, and 12 weeks following treatment by visual inspection of the retina and test of visual acuity. The affected areas of the retina appear healthy with no whitening (indicating lack of discernable retina damage) one week following PDT treatment; this trend continues throughout the monitoring period. Additionally, no evidence of optic nerve axon loss can been seen. Tests of visual acuity 4 and 12 weeks following combined PDT and PEDF treatment show no discernable loss of vision as a result of the treatment.

### Example 3

Brown Norway rats weighing 200-400 grams were treated intraperitoneally (i.p.) with either brimonidine, AGN 960, or the saline vehicle (not according to the invention). One hour later, the rats were unilaterally treated with PDT and evaluated by electroretinography 3-4 hours later. There were 4 rats in each treatment group.

PDT was conducted as follows: Mydriasis was induced in one eye of each rat with a drop of 0.5% tropicamide. Rats were then anesthetized with isoflurane and placed on a platform in front of a slit lamp coupled to a Coherent diode laser for verteporfin PDT (689 nm). Verteporfin was injected intravenously at a dose of 6 mg/m². One minute later, the retina of one eye was irradiated with a 3 mm size spot at 50 J/cm², 600 mW/cm² in the superior hemisphere above the optic disk. Three to four hours later, the treated animals were again dilated and anesthetized, then electroretinograms (ERGs) were evaluated in each eye.

ERGs are collected non-invasively by measuring mass-cell response arising due to retinal activity proceeding from a light stimulus. The first cells stimulated by a flash light stimulation are the photoreceptors at the outer retinal layer. This response is measured as an a-wave. As the signal is transduced to inner retinal neurons, a b-wave is produced. The a-wave reflects activity in the photoreceptors and the b-wave reflects activity in both photoreceptors and bipolar cells.

Before being subjected to the ERG test, the rats were placed in the dark (dark-adapted) for 15 minutes. Two types of ERG apparatus were used to generate the results below. In the initial brimonidine experiment, each eye was evaluated separately. A Grass photostimulator was placed 10 cm from the recording eye and flashed a single white flash lasting 10 microseconds. A gold corneal ring electrode with a reference electrode attached to the lower eyelid detected retinal responses. For the AGN 199960 experiments, a Ganzfeld dome was used to generate a single flash and sensitive bipolar corneal electrodes were placed on both eyes, thus allowing simultaneous ERGs to be done. A cushioning agent on the cornea (methylcellulose) was used in each case, and the responses were amplified and stored in a computer. The results reported are a-wave amplitudes, which appear to be the ERG parameter most affected in clinical PDT. As can be seen below, the loss in retinal function induced by PDT was inhibited by an alpha-2 receptor pan agonist (brimonidine)and a selective alpha-2B receptor agonist (AGN 960). AGN 960 has the following structure:

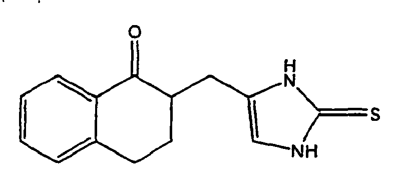

Brimonidine has the following structure:

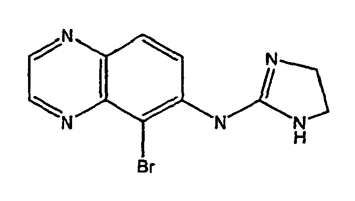

**Table 1. A-wave reflects photoreceptor function. Animals were treated with the indicated agent or vehicle i.p. 1 hour prior to PDT. * = p ≤ 0.05, comparison between drug and vehicle-treated animals utilizing an unpaired Student's t-test**

| | | | A Wave Amplitude | | |
|---|---|---|---|---|---|
| | | | (uV) | | |
| Agent | Dose | N | PDT Eye | Control Eye | % Control |
| Vehicle ** | 1 ml/kg | 4 | 70 ± 18 | 104 ± 17 | 67 ± 12 |
| Brimonidine | 1000 ug/kg | 4 | 90 ± 15 | 91 ± 21 | 103 ± 8 * |
| | | | | | |
| Vehicle ** | 1 ml/kg | 4 | 120 ± 12 | 228 ± 17 | 53 ± 3 |
| AGN 960 | 300 | 4 | 212 ± 16 | 218 ± 29 | 101 ± 13 |

| | | | | | |
|---|---|---|---|---|---|
| **) not according to the invention ug/kg | | | | | |

### Example 4 (not according to the invention)

Seven pigmented rabbits were dosed with either 0.5 mls of 0.2% brimonidine (alphagan) or saline administered retrobulbarly in 1 eye of each rabbit. One hour later, the animals were treated with a 10 minute intravenous infusion of 0.2 mg/kg verteporfin, then the same eye was irradiated 10 minutes later in the lower fundus with a 689 nm diode laser at 50 J/cm², 600 mW/cm² and a spot size of 1.5 mm.

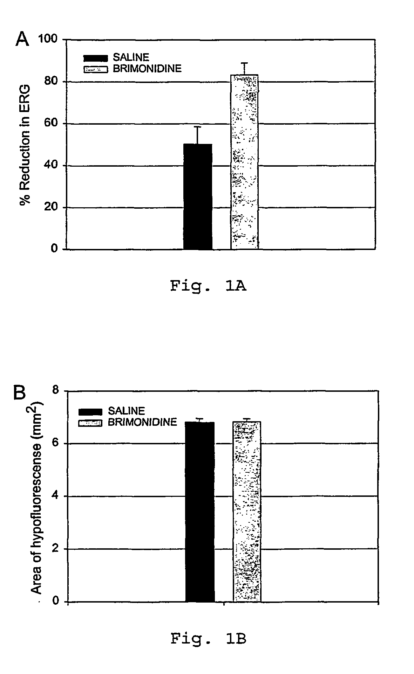

Treated eyes were then imaged by ocular coherence tomography; this method gives a measure of retinal thickness at the following time points after PDT (hours): 4, 24, 48, 72. Data are presented in Figure 1A and 1B; and show that brimonidine reduced the increase in retinal thickness (subretinal cyst + retina) in the lesion produced by PDT.

These examples illustrate certain embodiments of the present invention; however, it will be understood that the invention is solely defined by the claims that conclude this specification.

## Claims

1. Use of an alpha receptor agonist for the manufacture of a medicament for use in a method for reducing or eliminating a decrease in neurosensory retinal function following laser treatment of choroidal neovascularization (CNV) while maintaining the vascular occlusion therapeutic effect of such therapy, the method comprising the steps:
a) administering to a mammal having a CNV a therapeutically effective amount of said medicament containing an alpha receptor agonist wherein said neuroprotectant is selected from the group consisting of an alpha 2B selective agonist, alpha 2C selective agonist, clonidine and para-aminoclonidine.
b) subjecting said mammal to laser irradiation of the retinal locus of the CNV;
wherein the amount of neurosensory retinal function following steps a) and b) is greater than when said mammal is subjected to step b) without step a).

2. The use of claim 1 wherein prior to step b) said method comprises: administering to said patient a therapeutically effective amount of a photoactive agent in a manner such that said photoactive agent is present in the CNV during step b).

3. Use of a neuroprotectant compound for the manufacture of a medicament for use in a method of protecting ocular neural tissue from damage caused by electromagnetic irradiation of the retina comprising delivering to a patient's ocular neural tissue an amount of said medicament containing a neuroprotectant compound effective to protect a plurality of ocular neurons from cell death as compared to ocular neuron cell death following such irradiation observed in the absence of the administration of said neuroprotectant,
wherein said neuroprotectant is selected from the group consisting of an alpha 2B selective agonist, alpha 2C selective agonist, clonidine and para-aminoclonidine.

4. The use of claim 3 wherein said electromagnetic irradiation is laser irradiation.

5. The use of claim 1 or 3 wherein said alpha 2B and/or alpha 2C selective agonist is selected from the group consisting of AGN 960, AGN 7.95 and AGN 923.

6. The use of claim 5 in which the alpha 2B selective agonist is AGN 960.

7. The use of claim 5 in which the alpha 2B selective agonist is AGN 795.

8. The use of claim 5 in which the alpha 2B selective agonist is AGN 923.

9. The use of claim 3 wherein said neuroprotectant compound is administered at a time sufficiently before said electromagnetic irradiation to permit localization within ocular tissue prior to said treatment.

10. The use of claim 3 wherein said neuroprotectant compound is administered following said electromagnetic irradiation.

## Patentansprüche

1. Verwendung eines Alpha-Rezeptoragonisten zur Herstellung eines Medikaments zur Verwendung in einem Verfahren zum Vermindern oder Beseitigen einer Abnahme der neurosensorischen Retinalfunktion nach Laserbehandlung einer choroidalen Neovaskularisierung (CNV), während der therapeutische Gefäßverschlußeffekt einer solchen Therapie erhalten bleibt, wobei das Verfahren die Schritte umfaßt:
a) Verabreichen einer therapeutisch wirksamen Menge des Medikaments, das einen Alpha-Rezeptoragonisten enthält, an ein Säugetier mit einer CNV, wobei das Neuroschutzmittel ausgewählt ist aus der Gruppe bestehend aus einem Alpha-2B-selektiven Agonisten, Alpha-2C-selektiven Agonisten, Clonidin und para-Aminoclonidin,
b) das Säuretier einer Laserbestrahlung der retinalen Position der CNV unterziehen;
wobei die Menge der neurosensorischen Retinalfunktion nach den Schritten a) und b) größer ist als wenn das Säugetier dem Schritt b) ohne Schritt a) unterzogen wird.

2. Verwendung gemäß Anspruch 1, wobei das Verfahren vor dem Schritt b) das Verabreichen einer therapeutisch wirksamen Menge eines fotoaktiven Mittels an den Patienten in solcher Weise umfaßt, daß das fotoaktive Mittel während des Schritts b) in der CNV vorliegt.

3. Verwendung einer Neuroschutzmittelverbindung für die Herstellung eines Medikaments zur Verwendung in einem Verfahren zum Schützen des okularen Nervengewebes vor Beschädigung, die durch elektromagnetische Bestrahlung der Retina hervorgerufen wird, umfassend das Zuführen einer Menge des Medikaments, das eine Neuroschutzmittelverbindung enthält, die zum Schutz einer Vielzahl von okularen Neuronen vor dem Zelltod im Vergleich zu okularem Neuronenzelltod, der in der Folge solcher Bestrahlung in Abwesenheit der Verabreichung des Neuroschutzmittels beobachtet wird, wirksam ist, zum okularen Nervengewebe eines Patienten,
wobei das Neuroschutzmittel ausgewählt ist aus der Gruppe bestehend aus einem Alpha-2B-selektiven Agonisten, Alpha-2C-selektiven Agonisten, Clonidin und para-Aminoclonidin.

4. Verwendung gemäß Anspruch 3, wobei die elektromagnetische Strahlung Laserstrahlung ist.

5. Verwendung gemäß Anspruch 1 oder 3, wobei der Alpha-2Bund/oder Alpha-2C-selektive Agonist ausgewählt ist aus der Gruppe bestehend aus AGN 960, AGN 795 und AGN 923.

6. Verwendung gemäß Anspruch 5, in der der Alpha-2B-selektive Agonist AGN 960 ist.

7. Verwendung gemäß Anspruch 5, in der der Alpha-2B-selektive Agonist AGN 795 ist.

8. Verwendung gemäß Anspruch 5, in der der Alpha-2B-selektive Agonist AGN 923 ist.

9. Verwendung gemäß Anspruch 3, wobei die Neuroschutzmittelverbindung eine ausreichende Zeit vor der elektromagnetischen Bestrahlung verabreicht wird, um die Ansammlung innerhalb des Augengewebes vor der Behandlung zu erlauben.

10. Verwendung gemäß Anspruch 3, wobei die Neuroschutzmittelverbindung im Anschluß an die elektromagnetische Bestrahlung verabreicht wird.

## Revendications

1. Utilisation d'un agoniste de récepteur alpha pour la fabrication d'un médicament à utiliser dans un procédé de réduction ou d'élimination d'une diminution de la fonction rétinienne neurosensorielle après un traitement au laser de néovascularisation choroïdienne (CNV) tout en maintenant l'effet thérapeutique de l'occlusion vasculaire d'un tel traitement, le procédé comprenant les étapes suivantes :
a) administrer à un mammifère ayant une CNV une quantité thérapeutiquement efficace dudit médicament contenant un agoniste de récepteur alpha, dans lequel ledit neuro-protecteur est choisi dans le groupe constitué d'un agoniste sélectif 2B alpha, d'un agoniste sélectif 2C alpha, de clonidine et de para-aminoclonidine.
b) soumettre ledit mammifère à un rayonnement laser du locus rétinien de la CNV ; dans lequel l'ampleur de la fonction rétinienne neurosensorielle après les étapes a) et b) est supérieure à celle obtenue lorsque ledit mammifère est soumis à l'étape b) sans étape a).

2. Utilisation selon la revendication 1, dans laquelle, avant l'étape b), ledit procédé comprend : l'administration audit patient d'une quantité thérapeutiquement efficace d'un agent photo-actif de sorte que ledit agent photo-actif soit présent dans la CNV pendant l'étape b).

3. Utilisation d'un composé neuro-protecteur pour la fabrication d'un médicament à utiliser dans un procédé de protection du tissu nerveux oculaire contre les dommages causés par le rayonnement électromagnétique de la rétine, y compris la distribution au tissu nerveux oculaire d'un patient d'une quantité dudit médicament contenant un composé neuro-protecteur efficace pour protéger une pluralité de neurones oculaires de la mort cellulaire, par rapport à la mort cellulaire des neurones oculaires après ce rayonnement, observée en l'absence d'administration dudit composé neuro-protecteur, dans laquelle ledit composé neuro-protecteur est choisi dans le groupe constitué d'un agoniste sélectif alpha 2B, d'un agoniste sélectif alpha 2C, de clonidine et de para-aminoclonidine.

4. Utilisation selon la revendication 3, dans laquelle ledit rayonnement électromagnétique est un rayonnement laser.

5. Utilisation selon la revendication 1 ou 3, dans laquelle ledit agoniste sélectif alpha 2B et/ou alpha 2C est choisi dans le groupe constitué d'AGN 960, AGN 795 et AGN 923.

6. Utilisation selon la revendication 5, dans laquelle l'agoniste sélectif alpha 2B est l'AGN 960.

7. Utilisation selon la revendication 5, dans laquelle l'agoniste sélectif alpha 2B est l'AGN 795.

8. Utilisation selon la revendication 5, dans laquelle l'agoniste sélectif alpha 2B est l'AGN 923.

9. Utilisation selon la revendication 3, dans laquelle ledit composé neuro-protecteur est administré suffisamment tôt avant ledit rayonnement électromagnétique pour permettre la localisation dans le tissu oculaire avant ledit traitement.

10. Utilisation selon la revendication 3, dans laquelle ledit composé neuro-protecteur est administré après ledit rayonnement électromagnétique.
